# EUROPEAN PATENT APPLICATION

(11) **EP 3 064 935 A1**
(43) Date of publication of application: **07.09.2016**
(21) Application number: 15861357.0
(22) Date of filing: 29.09.2015
(51) Int. Cl.: G01N 27/404, A61B 1/12

(54) **CONCENTRATION METER AND ENDOSCOPE REPROCESSOR**

(30) Priority: 18.11.2014 JP 2014233872
(71) Applicant: Olympus Corporation, Hachioji-shi Tokyo 192-8507 (JP)
(72) Inventor: AKAHORI, Hiromasa, Tokyo 192-8507 (JP)
(74) Representative: Schicker, Silvia
(86) International application number: PCT/JP2015/077549
(87) International publication number: WO 2016/080077

(57) **Abstract**

A concentration meter of the invention includes: a main body section including an opening portion, and an internal liquid and an electrode housed in the opening portion; a permeation membrane that seals the opening portion; a water-holding section that is opposed to the permeation membrane with a predetermined distance separated from the permeation membrane; and an inflow/outflow section which is an opening that allows a clearance between the permeation membrane and the water-holding section to communicate with a space outside the main body section.

## Description

### Technical Field

The present invention relates to a concentration meter that measures a concentration of a solution and an endoscope reprocessor.

### Background Art

Endoscopes for use in medical fields are subjected to cleaning processing and disinfecting processing after use. In addition, endoscope reprocessors that automatically perform cleaning processing and disinfecting processing of endoscopes are known. In order to allow an endoscope reprocessor to have a function for automatically determining whether the use of a solution such as a disinfectant solution is permitted or not, a concentration meter for measuring the concentration of the solution is required.

For example, as disclosed in Japanese Patent Application Laid-Open Publication No. 2009-216523, a concentration meter is known, which is of a type using a permeation membrane that selectively allows the passage of certain gas or ion, and an electrode. When the concentration of a solution is measured with the concentration meter of the above-described type, a sensor section, which is a part where the permeation membrane is provided, is soaked in the solution.

In the concentration meter provided with a permeation membrane for measuring the concentration of the solution, the permeation membrane dehydrates when the sensor section is left in the air. When the permeation membrane is dry, a longer waiting time is required after the permeation membrane is soaked in the solution until a stable measurement result of the concentration of the solution can be obtained, compared with the case where the permeation membrane in a wet state. Therefore, in order to obtain a measurement result of the concentration of a solution in a shorter time with a concentration meter including a permeation membrane, it is necessary to keep the permeation membrane in a wet state even when the sensor section is placed in the air.

The present invention has been achieved in view of the above-described points, and an object of the present invention is to provide a concentration meter and an endoscope reprocessor that are capable of keeping a permeation membrane in a wet state for a long time even when a sensor section is placed in the air.

### Disclosure of Invention

### Means for Solving the Problem

A concentration meter according to one aspect of the present invention includes: a main body section including an opening portion, and an internal liquid and an electrode that are housed in the opening portion; a permeation membrane that seals the opening portion; a water-holding section that is opposed to the permeation membrane with a predetermined distance separated from the permeation membrane; and an inflow/outflow section which is an opening that allows a clearance between the permeation membrane and the water-holding section to communicate with an outside space of the main body section.

An endoscope reprocessor according to one aspect of the present invention includes: a storing container that stores a solution; a concentration meter fixed in the storing container, the concentration meter including a main body section including an opening portion and an internal liquid and an electrode housed in the opening portion, and a permeation membrane that seals the opening portion; and a water-holding section including an opposing face that is opposed to the permeation membrane with a predetermined distance separated from the permeation membrane, the water-holding section being fixed in the storing container such that an inflow/outflow section that allows a clearance between the permeation membrane and the opposing face to communicate with a space in the storing container is formed.

### Brief Description of the Drawings

FIG. 1 is a cross-sectional view showing a configuration of a sensor section of a concentration meter according to a first embodiment.
FIG. 2 is a perspective view of the sensor section according to the first embodiment.
FIG. 3 is a cross-sectional view taken along III-III line in FIG. 1.
FIG. 4 is a cross-sectional view showing a state where a solution is retained in a clearance in the sensor section according to the first embodiment.
FIG. 5 illustrates a modified example of a water-holding section according to the first embodiment.
FIG. 6 is a perspective view of a sensor section in a case where a float is located at a first position in a concentration meter according to a second embodiment.
FIG. 7 is a perspective view of a sensor section in a case where the float is located at a second position in the concentration meter according to the second embodiment.
FIG. 8 is a cross-sectional view of the sensor section in the case where the float is located at the first position in the concentration meter according to the second embodiment.
FIG. 9 is a cross-sectional view of the sensor section in the case where the float is located at the second position in the concentration meter according to the second embodiment.
FIG. 10 is a perspective view of the sensor section of the concentration meter according to a third embodiment.
FIG. 11 is a cross-sectional view of the sensor section of the concentration meter according to a third embodiment.
FIG. 12 illustrates a modified example of a water-holding section according to the third embodiment.
FIG. 13 illustrates a configuration of an endoscope reprocessor according to a fourth embodiment.
FIG. 14 illustrates a state where all of the solution is discharged from a storing container in the endoscope reprocessor according to the fourth embodiment.
FIG. 15 illustrates a configuration of a storing container of an endoscope reprocessor according to a fifth embodiment.

### Best Mode for Carrying Out the Invention

Hereinafter, preferred embodiments of the present invention will be described with reference to drawings. Note that, in the drawings used for describing the embodiments below, a different scale size is used for each of the constituent elements in order to allow each of the constituent elements to be illustrated in a recognizable size in the drawings, and the present invention is not limited only to the number, shapes, ratio of the sizes of the constituent elements, and a relative positional relationship among the constituent elements shown in these drawings.

Note that, in the description below, upper side indicates a position away from the ground with respect to an object to be compared, and lower side indicates a position close to the ground with respect to the object to be compared. In addition, the high and low relationship in the description below indicates a height relationship along the direction of gravitational force.

### (First Embodiment)

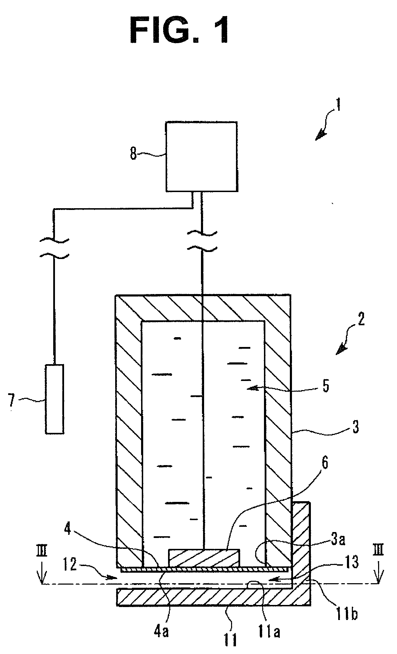

Hereinafter, description will be made on the first embodiment as one example of the embodiments of the present invention. A concentration meter 1 as shown in FIG. 1 includes a sensor section 2 to be soaked in a solution 10 (not shown in FIG. 1) when measuring the concentration of the solution 10.

The sensor section 2 includes a container-shaped main body section 3 having an opening portion 3 a. The opening portion 3 a is sealed by a permeation membrane 4. An internal liquid 5 and an electrode 6 are housed in the main body section 3 at a position more inside than the opening portion 3a. The permeation membrane 4 includes multiple perforations through which predetermined molecules in the solution pass. The material constituting the permeation membrane 4 is selected according to the type of the solution 10 as an object whose concentration is to be measured with the concentration meter 1, and is not limited in particular.

The electrode 6 and the permeation membrane 4 are in a connected state with the internal liquid 5. The word "connect" used here indicates that a state where a substance, as an object to be measured, which passes through the permeation membrane 4 to reach the internal liquid 5, can reach the electrode 6 with the internal liquid 5 as a medium.

The shape of an outer face 4a of the permeation membrane 4, which is a face to be in contact with the air outside of the main body section 3, is a planar shape in the present embodiment, as one example. Note that the shape of the outer face 4a of the permeation membrane 4 is not limited to the planar shape, but may be a curved face such as a cylindrical face or a spherical face. The outer face 4a can be restated as a part where the permeation membrane 4 contacts the solution 10 at the time of concentration measurement of the solution 10.

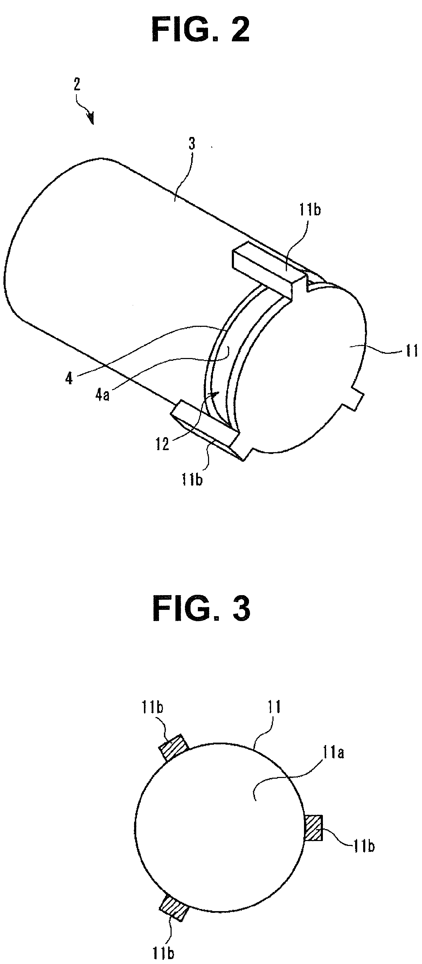

As shown in FIG. 2, the main body section 3 has a cylindrical shape, and the opening portion 3a is a circular-shaped bottomed hole portion formed on one end face of the main body section 3, as an example in the present embodiment. Therefore, the outer face 4a of the permeation membrane 4 that seals the circular-shaped opening portion 3 a has a circular shape. Note that the outer shape of the main body section 3 and the shape of the opening portion 3 are not limited to the shapes described in the present embodiment. For example, the main body section 3 may have a square columnar outer shape, for example. In addition, the opening portion 3a may be formed not on the end face but on a side face of the main body section 3, for example.

In addition, the opening portion 3 a may be formed in plurality on the main body section 3, for example. When a plurality of opening portions 3a are provided on the main body section 3, all the opening portions 3 a are sealed with one or a plurality of permeation membranes 4.

The electrode 6 is electrically connected to a control section 8. The concentration meter 1 includes a reference electrode 7 electrically connected to the control section 8, in addition to the sensor section 2. The position where the reference electrode 7 is disposed is determined according to the measurement principle used in the concentration meter 1, but not limited in particular. The reference electrode 7 may be disposed inside or outside of the main body section 3.

The concentration meter 1 measures, in the control section 8, a change in a potential difference generated between both of the electrodes, that is, the electrode 6 and the reference electrode 7, or a change in the value of the current flowing between both of the electrodes, and based on the measured value, measures the concentration of the solution 10 in which the sensor section 2 is soaked. Since such measurement principle and configuration of the electrochemical sensor are well-known, detailed description thereof will be omitted.

In addition, the concentration meter 1 includes a water-holding section 11. The water-holding section 11 is disposed at a position opposed to the outer face 4a of the permeation membrane 4. The position of the water-holding section 11 is fixed with respect to the permeation membrane 4. The water-holding section 11 includes an opposing face 11 a that is separated from the outer face 4a of the permeation membrane 4 by a predetermined distance and extended along the outer face 4a. In other words, the opposing face 11 a of the water-holding section 11 is a portion that covers the outer face 4a of the permeation membrane 4, with a predetermined distance separated from the outer face 4a of the permeation membrane 4.

As described above, the outer face 4a of the permeation membrane 4 has a planar shape, as one example in the present embodiment. Therefore, the shape of the opposing face 11 a of the water-holding section 11, which extends along the outer face 4a, has also a planar shape. For example, if the outer face 4a of the permeation membrane 4 is a cylindrical face convexed outward, the opposing face 11a of the water-holding section 11 is a cylindrical face concaved inward.

Note that the opposing face 11a may have a shape which covers the entirety of the outer face 4a of the permeation membrane 4 or a shape which covers a part of the outer face 4a of the permeation membrane 4. When the opposing face 11a has a shape which covers a part of the outer face 4a of the permeation membrane 4, it is preferable that the opposing face 11a has a shape which covers a region where the permeation membrane 4 and the opening portion 3a overlap with each other. In other words, it is preferable that the opposing face 11a is a member that covers at least the opening portion 3a when viewing the main body section 3 from an outer side.

Note that, when a plurality of opening portions 3a are provided, for example, the water-holding section 11 is disposed so as to cover all of the opening portions 3a. In this case, the water-holding section 11 may be formed by one member and configured to cover the entirety of the plurality of opening portions 3a, or configured to be divided into a plurality of members disposed so as to correspond respectively to the plurality of opening portions 3a.

As one example in the present embodiment, as shown in FIG. 2, the water-holding section 11 is a disk-shaped member, and disposed such that the opposing face 11 a, which is one face of the water-holding section 11, is opposed to the circular-shaped outer face 4a of the permeation membrane 4 and separated from the outer face 4a by a predetermined distance.

As described above, a clearance 13 having a width of the predetermined distance is formed between the outer face 4a of the permeation membrane 4 and the opposing face 11 a of the water-holding section 11. The clearance 13 communicates with the space around the sensor section 2 through an inflow/outflow section 12 which is one or a plurality of opening portions. The liquid including the solution 10 can flow into or flow out from the clearance 13 via the inflow/outflow section 12.

In the present embodiment, as one example, the main body section 3 and the water-holding section 11 are coupled with each other by one or a plurality of leg portions 11b that are disposed bridged over between the main body section 3 and the water-holding section 11, and the positions of the main body section 3 and the water-holding section 11 are fixed with respect to each other. The leg portion 11b couples the outer circumferential portion of the main body section 3 and the outer circumferential portion of the water-holding section 11. When there are plural leg portions 11b, the leg portions 11b are disposed so as to be separated from each other in a circumferential direction. In the present embodiment, the plural leg portions 11b are disposed at predetermined intervals in the circumferential direction, and the space between the leg portions 11b adjacent to each other serves as the inflow/outflow section 12 that allows communication between the clearance 13 and the space around the sensor section 2. Note that the inflow/outflow section 12 may be a hole portion which penetrates at least one of the main body section 3 and the water-holding section 11 and allows communication between the clearance 13 and the air around the sensor section 2, for example.

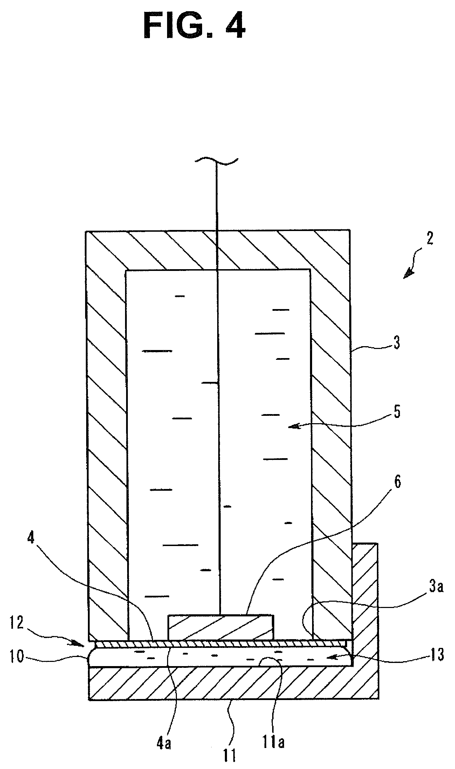

As shown in FIG. 4, the width of the clearance 13 between the outer face 4a of the permeation membrane 4 and the opposing face 11 a of the water-holding section 11 has a value set such that the solution 10 does not flow out from the inflow/outflow section 12 due to the surface tension of the solution and remains in the clearance 13 when the clearance 13 is filled with the solution 10 and thereafter the sensor section 2 is exposed to the air. That is, the width of the clearance 13 is defined depending on the surface tension of the solution 10. Thus, the width of the clearance 13 is not limited in particular, and defined appropriately depending on the viscosity of the solution 10 as an object to be measured, the wettability of the outer face 4a of permeation membrane 4, the wettability of the opposing face 11a of the water-holding section 11, and the like.

As described above, the concentration meter 1 according to the present embodiment includes, in the sensor section 2, the main body section 3 which includes the opening portion 3 a and houses the internal liquid 5 and the electrode 6, the permeation membrane 4 that seals the opening portion 3 a, the water-holding section 11 that is opposed to the permeation membrane 4 with the predetermined distance separated from the permeation membrane 4, and the inflow/outflow section 12 which allows the clearance 13 between the permeation membrane 4 and the water-holding section 11 to communicate with the air around the main body section 3.

When the concentration of the solution 10 is measured with the concentration meter 1 having the configuration as described above, the sensor section 2 is soaked in the solution 10. At this time, the solution 10 flows into the clearance 13 via the inflow/outflow section 12, which results in a contact between the outer face 4a of the permeation membrane 4 and the solution 10. After the solution 10 flows into the clearance 13, even if the sensor section 2 is exposed from the solution 10 to the air, the solution 10 continues to remain in the clearance 13 due to the surface tension, as shown in FIG. 4. The reason why the solution 10 remains in the clearance 13 even in the state where the sensor section 2 is exposed to the air is due to the action of surface tension of the solution 10. Therefore, even if the sensor section 2 is brought into a posture in which the inflow/outflow section 12 opens at a position on the lower side in the direction of gravitational force with respect to the clearance 13, the solution 10 remains in the clearance 13.

Therefore, the concentration meter 1 according to the present embodiment enables the permeation membrane 4 to be kept in a wet state with the solution 10 for a long time, even when the sensor section 2 is exposed to the air. Keeping the permeation membrane 4 in the wet state with the solution 10 can shorten the waiting time until the concentration measurement of the solution 10 becomes available again after the sensor section 2 is exposed from the solution 10 to the air.

At the time when the sensor section 2 is soaked in the solution 10, the solution 10 flows into the clearance 13 through the inflow/outflow section 12. As a result, the solution 10 retained in the clearance 13 in the state where the sensor section 2 is exposed to the air is discharged from the clearance 13. This prevents the solution retained in the clearance 13 in the state where the sensor section 2 is exposed to the air, i.e., old solution 10 from affecting the measurement result to be obtained with the concentration meter 1.

Note that, in order to retain the solution 10 in the clearance 13 in the state where the sensor section 2 is exposed to the air, it is preferable that the outer face 4a of the permeation membrane 4 has a hydrophilic property. The hydrophilic property of the outer face 4a of the permeation membrane 4 can be enhanced by performing hydrophilic treatment on the outer face 4a, for example. In addition, in order to retain the solution 10 in the clearance 13 in the state where the sensor section 2 is exposed to the air, it is preferable that the opposing face 11a of the water-holding section 11 has a hydrophobic property. The water-holding section 11 is made of polypropylene, for example, and silicone water repellent coating is applied to the opposing face 11a, to thereby capable of increasing the hydrophobic property of the opposing face 11a.

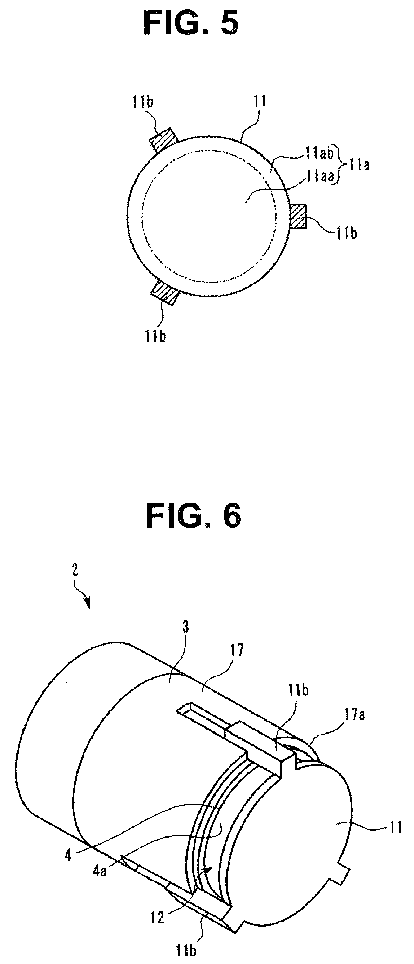

A modified example of the concentration meter 1 will be described with reference to FIG. 5. In the modified example shown in FIG. 5, the opposing face 11a of the water-holding section 11 is divided into two regions, i.e., a center portion 11aa and an outer circumferential portion 11ab surrounding the circumference of the center portion 11aa, and the outer circumferential portion 11ab is allowed to have a hydrophobic property stronger than that of the center portion 11aa. According to such a modified example, the solution 10 retained in the clearance 13 in the state where the sensor section 2 is exposed to the air gathers to the center portion 11aa, the hydrophobic property of which is weaker than that of the outer circumferential portion 11ab, which surely enables the permeation membrane 4 to be kept in the wet state.

### (Second Embodiment)

Next, description will be made on the second embodiment of the present invention. Only the points different from the first embodiment will be described below, the constituent elements same as those in the first embodiment are attached with the same reference numerals, and descriptions thereof will be appropriately omitted.

As shown in FIGS. 6 to 9, the concentration meter 1 according to the present embodiment is different from the one in the first embodiment in that a float 17 is provided to the sensor section 2. The float 17 has a specific gravity smaller than that of the solution 10 and larger than that of the air.

In the present embodiment, as one example, the float 17 is a cylindrical member that slides in an axial direction with respect to the main body section 3 on the outside of the main body section 3. The float 17 is capable of moving relatively with respect to the main body section 3 between a first position and a second position which are separated from each other in the axial direction of the main body section 3. The first position is on the upper side than the second position in the direction of gravitational force when the sensor section 2 is in such a posture that the end portion where a permeation membrane 4 is provided is directed to the lower side in the direction of gravitational force.

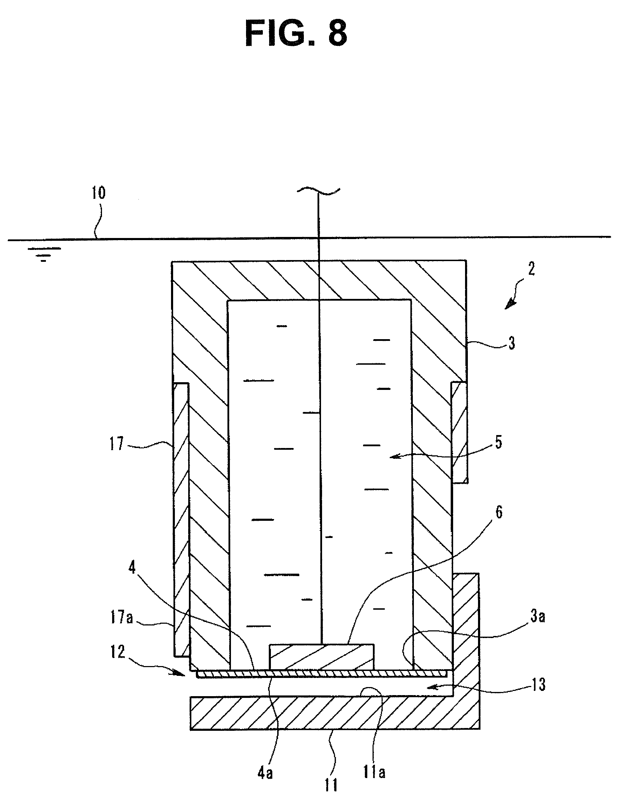

The float 17 moves to the first position with a buoyant force generated by the difference between the specific gravity of the float and the specific gravity of the solution 10, when the sensor section 2 is soaked in the solution 10 in such a posture that the end portion where the permeation membrane 4 is provided is directed to the lower side in the direction of gravitational force. FIG. 6 and FIG. 8 show the state where the float 17 is located at the first position.

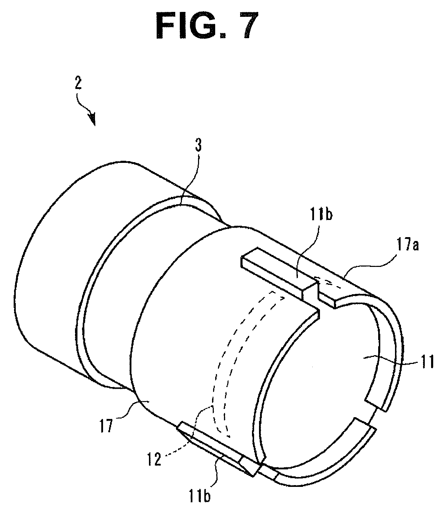

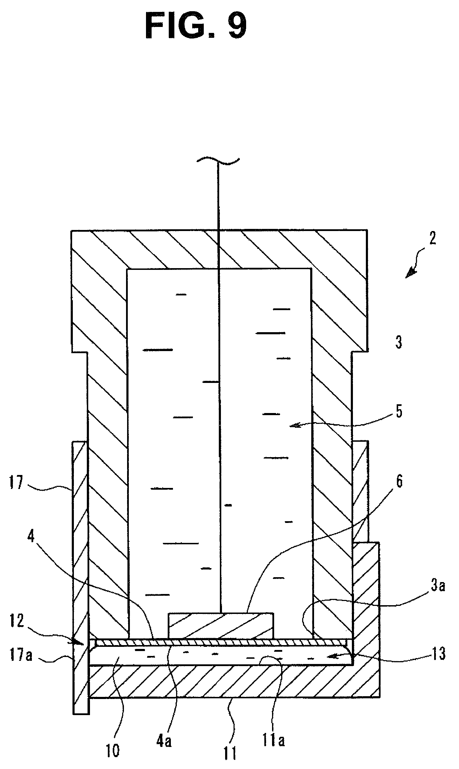

On the other hand, when the sensor section 2 is exposed to the air in the posture that the end portion where the permeation membrane 4 is provided is directed to the lower side in the direction of gravitational force, the float 17 moves to the second position by the self-weight. FIG. 7 and FIG. 9 show the state where the float 17 is located at the second position.

The float 17 includes a lid portion 17a which allows the inflow/outflow section 12 to be exposed outside the sensor section 2 when the float 17 is located at the first position, and which closes or narrows the inflow/outflow section 12 when the float 17 is located at the second position.

That is, the lid portion 17a opens the inflow/outflow section 12 when the sensor section 2 is soaked in the solution 10, and closes or narrows the inflow/outflow section 12 when the sensor section 2 is exposed to the air.

When the concentration of the solution 10 is measured with the concentration meter 1 thus configured according to the present embodiment, the sensor section 2 is soaked in the solution 10 in the posture that the end portion where the permeation membrane 4 is provided is directed to the lower side in the direction of gravitational force. At this time, the float 17 moves to the first position as shown in FIG. 6 and FIG. 8, to bring the inflow/outflow section 12 into the state of being exposed to the outside of the sensor section 2. Therefore, the solution 10 flows into the clearance 13 via the inflow/outflow section 12, and the solution 10 contacts the outer face 4a of the permeation membrane 4.

When the sensor section 2 is pulled out from the solution 10 to the air after the measurement of the concentration, the solution 10 continues to remain in the clearance 13 due to the surface tension, as described in the first embodiment. Furthermore, at this time, the float 17 moves to the second position by the self-weight, as shown in FIG. 7 and FIG. 9, and thereby the inflow/outflow section 12 is closed or narrowed by the lid portion 17a.

Therefore, the present embodiment is capable of preventing or suppressing the evaporation of the solution 10 retained in the clearance 13, when the sensor section 2 is exposed to the air, thereby enables the permeation membrane to be kept in the wet state for a longer time than in the first embodiment.

### (Third Embodiment)

Next, description will be made on the third embodiment of the present invention. Only the points different from the first embodiment will be described below, the constituent elements same as those in the first embodiment are attached with the same reference numerals, and descriptions thereof will be appropriately omitted.

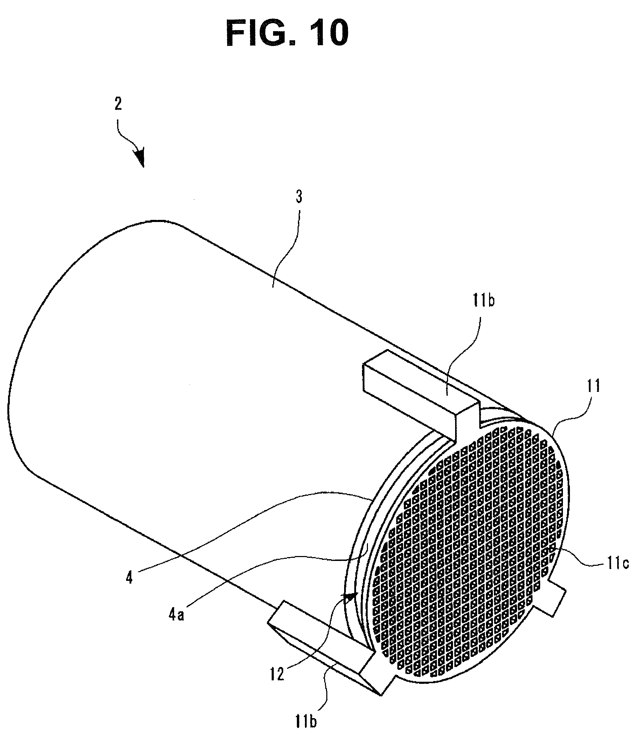

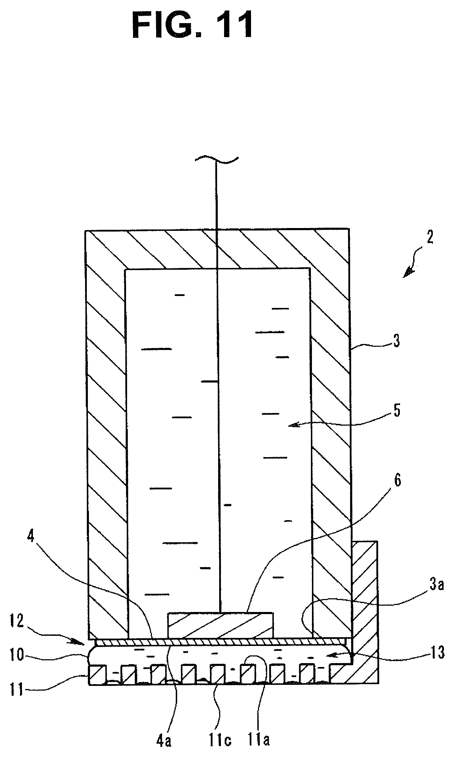

As shown in FIG. 10 and FIG. 11, the concentration meter 1 according to the present embodiment is different from the one in the first embodiment in that a mesh portion 11c is provided to the water-holding section 11. The water-holding section 11 according to the present embodiment is a plate-like member, and the mesh portion 11c is a part where a plurality of holes penetrating the water-holding section 11 in the thickness direction are formed.

Each of the holes formed on the mesh portion 11c has an inner diameter sized such that the solution 10 is retained in the through hole due to the surface tension of the solution, when the sensor section 2 is exposed to the air, as shown in FIG. 11.

With the concentration meter 1 having such a configuration according to the present embodiment, when the sensor section 2 is pulled out from the solution 10 to the air, the solution 10 continues to remain in the clearance 13 and in the holes of the mesh portion 11c due to the surface tension. Therefore, the concentration meter 1 according to the present embodiment is capable of keeping the permeation membrane 4 in the wet state with the solution 10 even in the case where the sensor section 2 is located in the air.

In the present embodiment, the water-holding section 11 disposed opposing to the outer face 4a of the permeation membrane 4 includes the mesh portion 11c including the plurality of holes that penetrate the water-holding section 11. Therefore, when the sensor section 2 exposed to the air is soaked into the solution 10, the solution 10 passes through not only the inflow/outflow section 12 but also the mesh portion 11c, to flow into the clearance 13. Accordingly, the solution 10 retained in the clearance 13 in the state where the sensor section 2 is exposed to the air is instantly replaced with the solution 10 that flows into the clearance 13. As a result, what is called the old solution 10 retained in the clearance 13 in the state where the sensor section 2 is exposed to the air is prevented from affecting the measurement result obtained with the concentration meter 1. For example, the concentration of the old solution 10 is likely to differ from the concentration of the solution 10 whose concentration is to be newly measured, due to the evaporation of the moisture in the air. However, the present embodiment is capable of surely preventing the old solution 10 from continuously being retained in the clearance 13 at the time of concentration measurement.

In order to retain the solution 10 in the clearance 13 in the state where the sensor section 2 is exposed to the air, it is preferable that the outer face 4a of the permeation membrane 4 has a hydrophilic property. In addition, in order to retain the solution 10 in the clearance 13 in the state where the sensor section 2 is exposed to the air, it is preferable that the mesh portion 11c has a hydrophilic property weaker than that of the outer face 4a of the permeation membrane 4, or has a hydrophobic property.

Note that the mesh portion 11c may be configured by providing the plurality of through holes on the plate-like water-holding section 11 as in the present embodiment, or may be configured by knitting a linear member made of metal or resin. In addition, the mesh portion 11c may be a porous member like a sponge. Furthermore, a part of or the entirety of the mesh portion 11c may contact the outer face 4a of the permeation membrane 4.

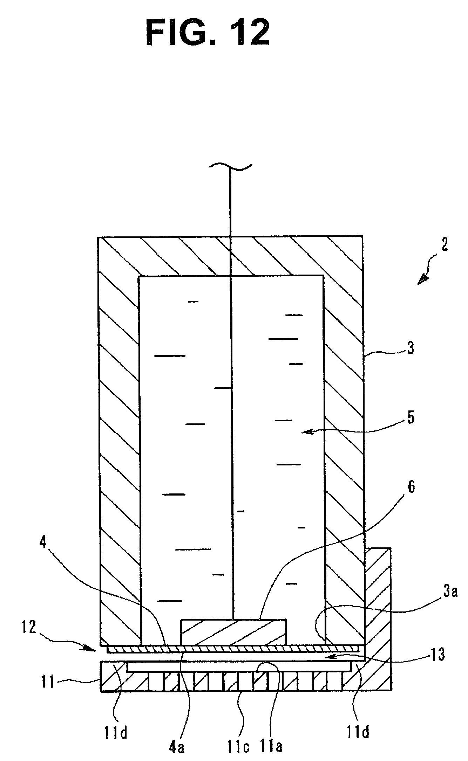

A modified example of the concentration meter 1 according to the present embodiment will be described with reference to FIG. 12. In the modified example shown in FIG. 12, an outer circumferential portion 11d that surrounds the mesh portion 11c is protruded toward the permeation membrane 4 side with respect to the mesh portion 11c. That is, the mesh portion 11c is surrounded by the wall-like outer circumferential portion 11d which protrudes toward the permeation membrane 4 side.

The wall protruding toward the permeation membrane 4 side is provided to the outer circumferential portion 11d of the mesh portion 11c as in the present modified example, thereby capable of surely retaining the solution 10 in the clearance 13 in the state where the sensor section 2 is exposed to the air.

### (Fourth Embodiment)

Next, description will be made on the fourth embodiment of the present invention. Only the points different from the first to third embodiments will be described below, the constituent elements same as those in the first to third embodiments are attached with the same reference numerals, and descriptions thereof will be appropriately omitted.

An endoscope reprocessor 20 according to the present embodiment includes the concentration meter 1 described in any one of the first to third embodiments. The endoscope reprocessor 20 is an apparatus that performs regeneration processing of a contaminated endoscope or endoscope accessories. The regeneration processing referred to here is not limited in particular, and may be any one of rinsing with water, cleaning for removing dirt such as organic substances, disinfecting for neutralizing a certain microorganism, sterilizing for eliminating or annihilating all microorganisms, or a combination of any of these.

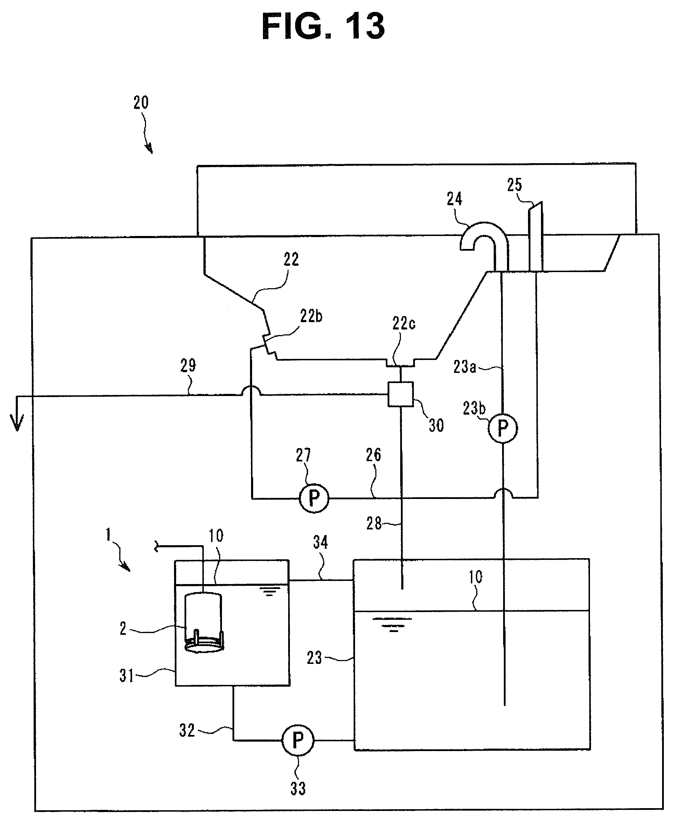

As shown in FIG. 13, the endoscope reprocessor 20 includes a processing basin 22 and a medicinal solution tank 23. The processing basin 22 has a recessed shape having an opening portion that opens upward, and is configured to be able to house therein at least one of the endoscope and endoscope accessories. The processing basin 22 is configured to be able to store liquid inside.

The medicinal solution tank 23 in the present embodiment is a part where the solution 10 as medicinal solution is stored. The medicinal solution may be any one of a cleaning solution to be used for cleaning, a disinfectant solution to be used for disinfection, and a sterilization solution to be used for sterilization. The solution to be stored in the medicinal solution tank 23 can include an aqueous peracetic acid solution as the disinfectant solution or the sterilization solution.

The medicinal solution tank 23 is connected to a medicinal solution nozzle 24 disposed in the processing basin 22, through a medicinal solution introducing conduit 23a. The medicinal solution introducing conduit 23a is provided with a medicinal solution introducing pump 23b. Activating the medicinal solution introducing pump 23b allows the solution 10 as the disinfectant solution stored in the medicinal solution tank 23 to be introduced into the processing basin 22.

In addition, a circulation nozzle 25 is disposed in the processing basin 22. The processing basin 22 includes at a lower portion thereof a circulation port 22b and a liquid drainage port 22c. The circulation nozzle 25 communicates with the circulation port 22b through a circulation conduit 26.

The circulation conduit 26 is provided with a circulation pump 27. Activating the circulation pump 27 allows the liquid in the processing basin 22 to be sucked out from the circulation port 22b and then to be returned to the processing basin 4 via the circulation conduit 26 and the circulation nozzle 25. The endoscope reprocessor 20 houses at least one of the endoscope and the endoscope accessories in the processing basin 22, to execute rinsing processing, reprocessing, and the like on at least one of the endoscope and the endoscope accessories by circulating water, medicinal solution, and the like.

The liquid drainage port 22c is a part that allows the liquid stored in the processing basin 22 to be discharged outside the processing basin 22 with the gravitational force. The liquid drainage port 22c is connected to a collection conduit 28, and a liquid drainage conduit 29, through a switching valve 30. The switching valve 30 is capable of switching between the state where the liquid drainage port 22c is opened and connected to either the collection conduit 28 or the liquid drainage conduit 29 and the state where the liquid drainage port 22c is closed.

The collection conduit 28 connects the switching valve 30 and the medicinal solution tank 23. If the liquid drainage port 22c is opened and the liquid drainage port 22c is connected to the liquid drainage conduit 29 in the state where the solution 10 as the disinfectant solution is stored in the processing basin 22, the solution 10 in the processing basin 22 is collected into the medicinal solution tank 23.

The liquid drainage conduit 29 is extended outside the endoscope reprocessor 20. If the liquid drainage port 22c is opened and connected to the liquid drainage conduit 29, the liquid stored in the processing basin 22 is discharged outside the endoscope reprocessor 20.

In addition, the endoscope reprocessor 20 includes therein a storing container 31 in which the sensor section 2 of the concentration meter 1 is housed. The storing container 31 communicates with the medicinal solution tank 23 through a conduit 32 and a return conduit 34. The conduit 32 is provided with a pump 33. Operation of the pump 33 causes the solution 10 in the medicinal solution tank 23 to circulate so as to pass through the conduit 32, the storing container 31, and the return conduit 34, and then return to the medicinal solution tank 23. Note that if the pump 33 is configured to be operable in normal and reverse directions, even if the return conduit 34 is omitted, it is possible to perform operation for transferring the solution 10 in the medicinal solution tank 23 to the storing container 31 via the conduit 32 and operation for transferring the solution 10 in the storage tank 31 to the medicinal solution tank 23 via the conduit 32.

Note that either the inflow of the solution 10 into the storing container 31 or the discharge of the solution 10 from the storing container 31 may be performed by the working of the gravitational force. In addition, the inflow of the solution 10 into the storing container 31 and the discharge of the solution 10 from the storing container 31 may be performed respectively via different conduits. Furthermore, the storing container 31 may be provided with a conduit for liquid drainage which is extended outside the apparatus.

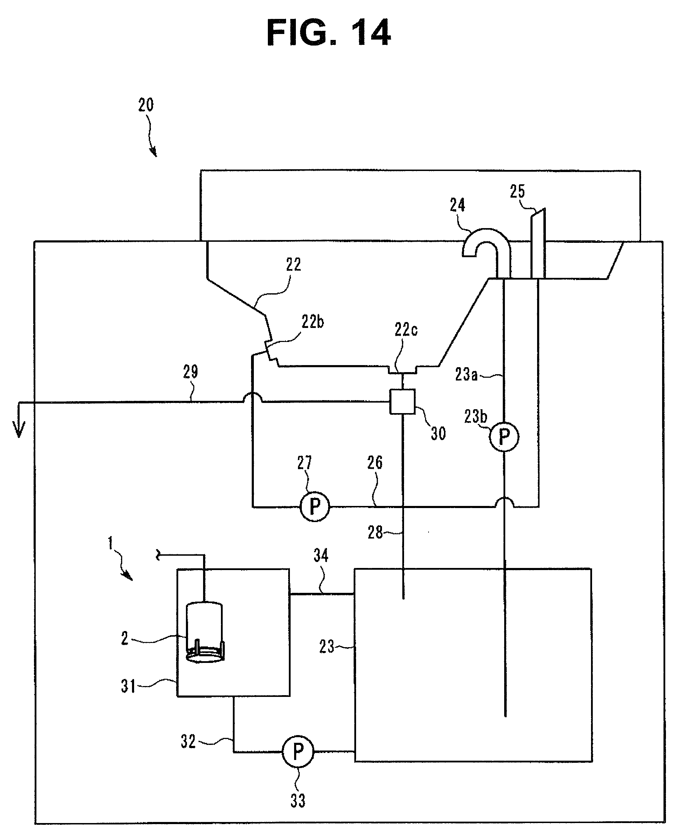

In the endoscope reprocessor 20 according to the present embodiment, when rinsing processing, disinfecting processing and the like are performed on at least one of the endoscope and the endoscope accessories in the processing basin 22, all of the solution 10 is discharged from the storing container 31, as shown in FIG. 14. In addition, also when the solution 10 is discharged from the medicinal solution tank 23 in order to exchange the disinfectant solution, for example, all of the solution 10 is discharged from the storing container 31.

Then, the endoscope reprocessor 20 measures the concentration of the solution 10 stored in the medicinal solution tank 23 before the disinfecting processing and the like are performed on at least one of the endoscope and endoscope accessories. When the measurement of the concentration of the solution 10 is performed, the endoscope reprocessor 20 activates the pump 33, to allow the solution 10 of a predetermined volume to flow from the medicinal solution tank 23 into the storing container 31, as shown in FIG. 13. The volume of the solution 10 transferred to the storing container 31 is supposed to have a value set such that the sensor section 2 is soaked in the solution.

After the solution 10 flows into the storing container 31, the endoscope reprocessor 20 activates the concentration meter 1 to measure the concentration of the solution 10. When the measurement result of the concentration of the solution 10 obtained with the concentration meter 1 is within a predetermined value range, the endoscope reprocessor 20 determines that reprocessing with the use of the solution 10 can be performed on at least one of the endoscope and the endoscope accessories. On the other hand, when the measurement result of the concentration of the solution 10 obtained with the concentration meter 1 is not within a predetermined value range, the endoscope reprocessor 20 determines that reprocessing with the use of the solution 10 cannot be performed on at least one of the endoscope and the endoscope accessories, and issues an alert to a user by emitting sound or light, for example.

After the concentration measurement of the solution 10 has been performed with the concentration meter 1, the endoscope reprocessor 20 activates the pump 33 to transfer all of the solution 10 in the storing container 31 to the medicinal solution tank 23. At this time, as shown in FIG. 14, the sensor section 2 of the concentration meter 1 is brought into a state exposed to the air as shown in FIG. 14.

As described above, in the endoscope reprocessor 20, the sensor section 2 of the concentration meter 1 is soaked in the solution 10 only during the period in which the concentration measurement is executed. In other words, in the endoscope reprocessor 20, the state where the sensor section 2 is soaked in the solution 10 and the state where the sensor section 2 is exposed to the air are repeated.

As described in the first to third embodiments, the concentration meter 1 is capable of keeping the permeation membrane 4 in the wet state with the solution 10 for a long time in the state where the sensor section 2 is exposed to the air. Therefore, the endoscope reprocessor 20 according to the present embodiment can reduce the waiting time until the measurement of the concentration is started after the solution 10 is transferred into the storing container 31 and the sensor section 2 is soaked in the solution 10. That is, according to the present embodiment, the time required for executing the concentration measurement of the solution 10 as the disinfectant solution can be reduced, thereby capable of increasing the number of endoscopes which are subjected to the disinfecting processing per unit time.

Note that the sensor section 2 may be disposed in the medicinal solution tank 23, and the concentration meter 1 may be configured to directly measure the concentration of the solution 10 in the medicinal solution tank 23. That is, the medicinal solution tank 23 and the storing container 31 may be the same container.

In addition, the sensor section 2 according to the present embodiment may include the float 17 described in the second embodiment and a mesh portion 11c described in the third embodiment.

### (Fifth Embodiment)

Next, description will be made on the fifth embodiment of the present invention. Hereinafter, only the points different from the fourth embodiment will be described. The constituent elements same as those in the fourth embodiment are attached with the same reference numerals, and descriptions thereof will be appropriately omitted.

In the above-described fourth embodiment, the water-holding section 11 of the concentration meter 1 is fixed to the sensor section 2. The present embodiment is different from the fourth embodiment in that the water-holding section 11 is fixed to the storing container 31.

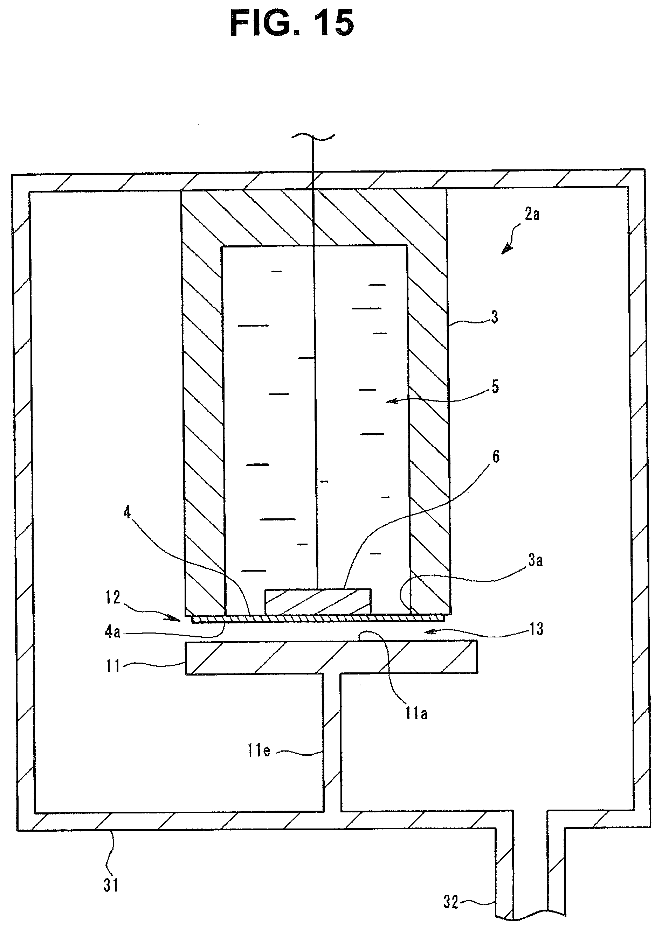

As shown in FIG. 15, a sensor section 2a of the concentration meter according to the present embodiment includes the main body section 3 including an opening portion 3a and housing the internal liquid 5 and the electrode 6, and the permeation membrane 4 that seals the opening portion 3a. The sensor section 2 is fixed in the storing container 31.

Inside the storing container 31, the water-holding section 11 disposed so as to be opposed to the outer face 4a of the permeation membrane 4 is fixed. That is, the position of the water-holding section 11 is fixed with respect to the permeation membrane 4. In the present embodiment, as one example, the water-holding section 11 is held in the storing container 31 with leg portions 11e disposed so as to be bridged between the wall surface of the storing container 31 and the water-holding section 11. The water-holding section 11 includes an opposing face 11a separated from the outer face 4a of the permeation membrane 4 by a predetermined distance and extended along the outer face 4a. Note that the water-holding section 11 may be a part of the wall surface of the storing container 31.

Also in the present embodiment, similarly as in the concentration meter 1 according to the first to third embodiments, the clearance 13 having a width of the predetermined distance is formed between the outer face 4a of the permeation membrane 4 and the opposing face 11a of the water-holding section 11. In addition, since the water-holding section 11 is fixed at a position separated from the outer face 4a of the permeation membrane 4, the inflow/outflow section 12 that allows communication between the clearance 13 and the space in the storing container 31 is formed around the clearance 13. The width of the clearance 13 has a value set such that the solution 10 does not flow out from the inflow/outflow section 12 and remains in the clearance 13 due to the surface tension of the solution when the sensor section 2 is placed in the air after the clearance 13 is filled with the solution 10.

Therefore, the endoscope reprocessor 20 according to the present embodiment is capable of keeping the permeation membrane 4 in the wet state with the solution 10 for a long time, in the state where the sensor section 2 is exposed to the air, similarly as in the fourth embodiment. Therefore, the endoscope reprocessor 20 according to the present embodiment can reduce the waiting time until the measurement of the concentration is started after the solution 10 is transferred into the storing container 31 and the sensor section 2 is soaked in the solution 10. That is, according to the present embodiment, the time required for executing the concentration measurement of the solution 10 as the disinfectant solution can be reduced, thereby capable of increasing the number of endoscopes which are subjected to the disinfecting processing per unit time.

Note that the sensor section 2a and the water-holding section 2 may be disposed in the medicinal solution tank 23, and the concentration meter 1 may be configured to directly measure the concentration of the solution 10 in the medicinal solution tank 23. That is, the medicinal solution tank 23 and the storing container 31 may be the same container.

In addition, the sensor section 2 according to the present embodiment may include the float 17 described in the second embodiment and the mesh portion 11c described in the third embodiment.

Note that the present invention is not limited to the above-described embodiments, and can be properly changed within the range without departing from the gist or the idea of the invention that can be read from claims and the entire description, and a concentration meter and an endoscope reprocessor accompanied by such changes are also included in the technical range of the present invention.

The sensor section 2 according to the present invention can be applied to an apparatus that detects a component other than the disinfectant solution. For example, the sensor section 2 can be applied to an apparatus that detects oxygen concentration or pH level in a solution.

Furthermore, in the above-described first to fifth embodiments, the sensor section 2 has a configuration of what is generally called an electrochemical sensor, but the sensor section 2 may be a measurement section of another configuration, for example, an absorbance sensor. In addition, the sensor section 2 may be configured as a gas detection sensor in which the main body section 3 is filled with gas instead of the internal liquid 5, for example. The gas includes low active gas such as air, nitrogen, or noble gas.

The present application claims the benefit of Japanese Patent Application No. 2014-233872 filed in Japan on November 18, 2014, the entire disclosure contents of which are incorporated in the description, claims and drawings of the present application by reference.

## Claims

1. A concentration meter comprising:
a main body section including an opening portion, and an internal liquid and an electrode that are housed in the opening portion;
a permeation membrane that seals the opening portion;
a water-holding section that is opposed to the permeation membrane with a predetermined distance separated from the permeation membrane; and
an inflow/outflow section which is an opening that allows a clearance between the permeation membrane and the water-holding section to communicate with an outside space of the main body section.

2. The concentration meter according to claim 1, wherein
an opposing face of the water-holding section, which is opposed to the permeation membrane, includes two regions, the two regions being a center portion and an outer circumferential portion surrounding the center portion, and
the outer circumferential portion has a hydrophobic property stronger than a hydrophobic property of the center portion.

3. The concentration meter according to claim 1 or 2, further comprising a float having a specific gravity smaller than a specific gravity of a solution as an object to be measured with the concentration meter and larger than a specific gravity of air,
wherein the float is capable of relatively moving between a first position and a second position with respect to the main body section, the float moves to the first position by a buoyant force and allows the inflow/outflow section to be exposed to an outside of the main body section when the permeation membrane is soaked in the solution, and the float moves to the second position by a self-weight of the float and closes or narrows the inflow/outflow section when the permeation membrane is exposed to the air.

4. An endoscope reprocessor comprising:
a storing container that stores a solution;
a concentration meter fixed in the storing container, the concentration meter comprising a main body section including an opening portion and an internal liquid and an electrode housed in the opening portion, and a permeation membrane that seals the opening portion; and
a water-holding section including an opposing face that is opposed to the permeation membrane with a predetermined distance separated from the permeation membrane, the water-holding section being fixed in the storing container such that an inflow/outflow section that allows a clearance between the permeation membrane and the opposing face to communicate with a space in the storing container is formed.
